(19) Europäisches Patentamt
European Patent Office
Office européen des brevets

(11) **EP 4 289 808 A1**

(12) **EUROPEAN PATENT APPLICATION**
published in accordance with Art. 153(4) EPC

(43) Date of publication:
**13.12.2023 Bulletin 2023/50**

(21) Application number: **22749919.1**

(22) Date of filing: **24.01.2022**

(51) International Patent Classification (IPC):
$C07C\ 31/27^{(2006.01)}$     $C07C\ 29/32^{(2006.01)}$
$C07C\ 29/149^{(2006.01)}$     $B01J\ 23/44^{(2006.01)}$

(52) Cooperative Patent Classification (CPC):
**B01J 23/44; C07C 29/149; C07C 29/32;
C07C 31/27**

(86) International application number:
**PCT/KR2022/001257**

(87) International publication number:
**WO 2022/169165 (11.08.2022 Gazette 2022/32)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **03.02.2021 KR 20210015384
03.02.2021 KR 20210015385**

(71) Applicant: **Hanwha Solutions Corporation
Jung-gu
Seoul 04541 (KR)**

(72) Inventors:
• **KIM, Eun Jeong
  Daejeon 34128 (KR)**
• **LEE, Sun Uk
  Daejeon 34128 (KR)**
• **JANG, Namjin
  Daejeon 34128 (KR)**

(74) Representative: **Berggren Oy
P.O. Box 16
Eteläinen Rautatiekatu 10A
00101 Helsinki (FI)**

(54) **1,4-CYCLOHEXANEDIMETHANOL COMPOSITION AND PURIFICATION METHOD THEREFOR**

(57) The present disclosure relates to a 1,4-cyclohexanedimethanol (CHDM) composition and a purification method of the same. More specifically, it relates to a 1,4-cyclohexanedimethanol composition having a high purity due to a low by-product content and a high trans isomer ratio, and a method for purifying the same.

**EP 4 289 808 A1**

**Description**

[TECHNICAL FIELD]

Cross-reference to Related Application

This application claims the benefits of Korean Patent Applications No. 1 0-2021-0015384 filed on February 3, 2021 and No. 10-2021-0015385 filed on February 3, 2021 with the Korean Intellectual Property Office, the disclosure of which is incorporated herein by reference in its entirety.

The present disclosure relates to a method for purifying a 1,4-cyclohexanedimethanol (CHDM) composition. More specifically, it relates to a 1,4-cyclohexanedimethanol composition having a low by-product content, a high purity and a high trans isomer ratio, and a method for purifying the same.

[BACKGROUND OF ART]

1,4-Cyclohexanedimethanol (CHDM) is widely used as a raw material for medicines, synthetic resins, synthetic fibers, or dyes, and particularly, is used as a raw material for polyethylene terephthalate, which is an environmentally friendly polyester.

1,4-Cyclohexanedimethanol exists as stereoisomers of cis- and transforms, and higher quality products require a higher ratio of trans 1,4-cyclohexanedimethanol (trans CHDM) than cis CHDM.

Among the methods for producing 1,4-cyclohexanedimethanol, a method of hydrogenating dimethyl terephthalate (DMT) is widely used commercially. In this method, DMT is prepared by reacting phthalate with methanol, and then 1,4-cyclohexanedimethanol is produced by a two-step hydrogenation reaction. The first hydrogenation reaction converts DMT into diester dimethyl 1,4-cyclohexanedicarboxylate (DMCD), and the second hydrogenation reaction converts DMCD into CHDM. At this time, the ratio of cis CHDM and trans CHDM is determined according to the type of catalyst. When using a copper chromite catalyst, which is commercially used copper chromium oxide, the ratio of cis CHDM and trans CHDM is about 3:7. Since this method uses DMT and trans esterification with methanol, the reaction and separation process are complicated, and additives must be used for isomerization, which can affect the quality of final products.

[DETAILED DESCRIPTION OF THE INVENTION]

[Technical Problem]

In order to solve the above problems, there is provided 1,4-cyclohexanedimethanol having a high purity due to a low content of by-products and water, and a high trans isomer ratio by purifying a 1,4-cyclohexanedimethanol crude composition.

[Technical Solution]

According to an aspect of the present disclosure, there is provided a 1,4-cyclohexanedimethanol composition including 1,4-cyclohexanedimethanol (CHDM) having a purity of 99.7 wt% or more as measured by gas chromatography (GC) and containing cis isomers and trans isomers; and 0.15 wt% or less of a light by-product having a molecular weight of less than 144.21 g/mol.

According to another aspect of the present disclosure, there is provided a method for purifying a 1,4-cyclohexanedimethanol composition, including the steps of:

a water removal step of removing water from a 1,4-cyclohexanedimethanol (CHDM) crude composition;
a first by-product removal step of removing a by-product having a lower boiling point than 1,4-cyclohexanedimethanol from the 1,4-cyclohexanedimethanol composition that has undergone the water removal step; and
a second by-product removal step of recovering a purified 1,4-cyclohexanedimethanol composition by removing a by-product having a higher boiling point than 1,4-cyclohexanedimethanol from the 1,4-cyclohexanedimethanol composition that has undergone the first by-product removal step.

[ADVANTAGEOUS EFFECTS]

According to the 1,4-cyclohexanedimethanol composition and its purification method of the present disclosure, it is possible to provide 1,4-cyclohexanedimethanol with a high purity due to a very low content of by-products and water, and improve physical properties when used as a polymer raw material.

In addition, the method for purifying 1,4-cyclohexanedimethanol of the present disclosure consumes little energy, has simple process steps, and has high by-product removal efficiency, so that high-quality 1,4-cyclohexanedimethanol can be produced at low cost.

[DETAILED DESCRIPTION OF THE EMBODIMENTS]

The terminology used herein is for the purpose of describing particular embodiments only and is not intended to be limiting of the invention. The singular forms are intended to include the plural forms as well, unless the context clearly indicates otherwise. It will be further understood that the terms "include", "comprise", or "have" when used in this disclosure, specify the presence of stated features, steps, components, or combinations thereof, but do not preclude the presence or addition of one or more other features, steps, components, or combinations thereof.

As the present invention can be variously modified and have various forms, specific embodiments thereof are shown by way of examples and will be described in detail. However, it is not intended to limit the present invention to the particular form disclosed and it should be understood that the present invention includes all modifications, equivalents, and replacements within the idea and technical scope of the present invention.

Hereinafter, the method for purifying a 1,4-cyclohexanedimethanol composition according to specific embodiments of the present disclosure will be described in more detail.

The method for purifying 1,4-cyclohexanedimethanol of the present disclosure includes the steps of: a water removal step of removing water from a 1,4-cyclohexanedimethanol (CHDM) crude composition; a first by-product removal step of removing a by-product having a lower boiling point than 1,4-cyclohexanedimethanol from the 1,4-cyclohexanedimethanol composition that has undergone the water removal step; and a second by-product removal step of recovering a purified 1,4-cyclohexanedimethanol composition by removing a by-product having a higher boiling point than 1,4-cyclohexanedimethanol from the 1,4-cyclohexanedimethanol composition that has undergone the first by-product removal step.

Hereinafter, the 1,4-cyclohexanedimethanol composition and the purification method thereof according to an embodiment of the present invention will be described in detail for each step.

The 1,4-cyclohexanedimethanol composition of the present disclosure includes 1,4-cyclohexanedimethanol (CHDM) having a purity of 99.7 wt% or more as measured by gas chromatography (GC) and containing cis isomers and trans isomers; and 0.15 wt% or less of a light by-product having a molecular weight of less than 144.21 g/mol.

More specifically, the 1,4-cyclohexanedimethanol composition according to an embodiment of the present disclosure may include a light by-product having a molecular weight (Mw) of less than 144.21 g/mol in an amount of 0.15 wt% or less, 0.14 wt% or less, 0.12 wt% or less, 0.10 wt% or less, or 0.09 wt% or less. As the lower content of the light by-product can be evaluated as the better, the lower limit may be 0 wt%, for example, 0.001 wt%.

In the case of producing another polymer using the 1,4-cyclohexanedimethanol composition including more than 0.15 wt% of the light by-product, it may be difficult to manufacture a high molecular weight and high quality product due to hindered polymerization of the polymer.

Examples of the light by-product may include cyclohexylmethanol, 4-methyl-1-cyclohexanemethanol, cyclohexane carboxylic acid, 4-methyl-3-cyclohexene-1-methanol, 4-methyl cyclohexane carboxylic acid, 4-hydroxymethyl cyclohexane carboxylic acid, 4-hydroxymethyl-4-cyclohexanemethanol, 3-hydroxymethyl-4-cyclohexanemethanol, and the like.

When a large amount of cyclohexylmethanol (CHM) among the above-described light by-products is included in the final product, the distribution of the light polymer increases during polymer production, which affects intrinsic properties of the polymer such as the molecular weight distribution and intrinsic viscosity. Therefore, it is preferable to remove cyclohexylmethanol as much as possible and to include substantially no cyclohexylmethanol.

The 1,4-cyclohexanedimethanol composition according to an embodiment of the present disclosure includes only a very small amount of 0.01 wt% or less, or 0.005 wt% or less or only an undetectable amount of the cyclohexylmethanol, so that it can be used as a raw material for high-quality polymer products.

In addition, in the 1,4-cyclohexanedimethanol composition according to an embodiment of the present disclosure, a heavy by-product having a molecular weight of more than 144.21 g/mol, which is the molecular weight (Mw) of 1,4-cyclohexanedimethanol, may be included in an amount of 0.15 wt% or less, 0.14 wt% or less, 0.12 wt% or less, 0.10 wt% or less, or 0.09 wt% or less based on the total weight of the purified 1,4-cyclohexanedimethanol composition. As the lower content of the heavy by-product can be evaluated as the better, the lower limit may be 0 wt%, for example, 0.01 wt%.

In the case of producing another polymer using the 1,4-cyclohexanedimethanol composition including more than 0.15 wt% of the heavy by-product, the carboxyl group at the end of the heavy by-product hinders polymerization of the polymer, making it difficult to prepare a polymer having a desired molecular weight and causing yellowing. Thus, it may be difficult to manufacture high-quality products.

Examples of the heavy by-product may include 1,4-cyclohexane-hydroxylmethyl-carboxylic acid, a monoester-based compound, or a monoether-based compound.

In addition, the 1,4-cyclohexanedimethanol composition according to an embodiment of the present disclosure may have an APHA value of 10 or less, 8 or less, 6 or less, 4 or less, or 2 or less, when measured according to ASTM D1209, indicating excellent color.

In addition, in the 1,4-cyclohexanedimethanol composition according to an embodiment of the present disclosure, the 1,4-cyclohexanedimethanol may contain the trans isomer, i.e., trans 1,4-cyclohexanedimethanol in an amount of 63 wt% or more, 65 wt% or more, 67 wt% or more, 69 wt% or more, or 70 wt% or more, indicating a very high trans isomer content. Further, there is no upper limit on the trans isomer ratio, but it may be, for example, 99 wt% or less, 95 wt% or less, 90 wt% or less, or 85 wt% or less.

In addition, in the 1,4-cyclohexanedimethanol composition according to an embodiment of the present disclosure, the content of 1,4-cyclohexanedimethanol, i.e., the purity may be 99.5 wt% or more, 99.6 wt% or more, 99.7 wt% or more, or 99.75 wt% or more. As the higher purity can be evaluated as the better, the theoretical purity is 100 wt%, but may be substantially 99.9 wt% or less.

As described above, since the 1,4-cyclohexanedimethanol composition according to an embodiment of the present disclosure has a high 1,4-cyclohexanedimethanol purity, a high trans content, and a very low content of residual by-products, it can be usefully used as a raw material for manufacturing a high-quality product such as pharmaceuticals, synthetic resins, synthetic fibers, or dyes.

The 1,4-cyclohexanedimethanol composition of the present disclosure having the above characteristics may be obtained, for example, by a purification method including the steps of: a water removal step of removing water from a 1,4-cyclohexanedimethanol (CHDM) crude composition; a first by-product removal step of removing a by-product having a lower boiling point than 1,4-cyclohexanedimethanol from the 1,4-cyclohexanedimethanol composition that has undergone the water removal step; and a second by-product removal step of recovering a purified 1,4-cyclohexanedimethanol composition by removing a by-product having a higher boiling point than 1,4-cyclohexanedimethanol from the 1,4-cyclohexanedimethanol composition that has undergone the first by-product removal step.

Hereinafter, the method for purifying a 1,4-cyclohexanedimethanol composition according to an embodiment of the present disclosure will be described in detail for each step.

First, a step of removing water is performed on a 1,4-cyclohexanedimethanol crude composition including 1,4-cyclohexanedimethanol (CHDM), water, and by-products.

The 1,4-cyclohexanedimethanol crude composition, which is the subject of purification in the purification method of the present disclosure, is obtained by, but is not limited to, a two-step hydrogenation reaction of terephthalic acid. Since the terephthalic acid, which is a first reactant, undergoes a hydrogenation reaction while dissolved in water, the 1,4-cyclohexanedimethanol crude composition contains a large amount of water.

For example, the 1,4-cyclohexanedimethanol crude composition includes 10 to 40 wt% of 1,4-cyclohexanedimethanol, 60 to 90 wt% of water, and by-products based on the total weight. Water occupies most of the remaining components except for 10 to 40 wt% of 1,4-cyclohexanedimethanol.

Accordingly, it is necessary to first remove water in order to recover highly purified 1,4-cyclohexanedimethanol. However, since the 1,4-cyclohexanedimethanol crude composition contains a large amount of water as described above, a lot of energy is consumed when water is also removed in the by-product removal step.

Therefore, in an embodiment of the present disclosure, first, a water removal step of removing a large amount of water is performed in a low temperature and high vacuum state.

For example, the water removal step may be performed at a temperature of 50 to 110 °C and a pressure of -0.1 to 0.1 barg on the 1,4-cyclohexanedimethanol crude composition. More preferably, it may be performed at a temperature of 55 to 105 °C and a pressure of 0 to 0.01 barg.

When the temperature and pressure conditions are out of the above-described conditions, water is not sufficiently removed, and an additional water removal step is required in a subsequent process, which may increase energy consumption required for purification.

The water removal step may be performed using an apparatus such as an evaporator, a multi-stage evaporator, or a distillation column, but the present disclosure is not limited thereto.

After the above-described water removal step, 80 wt% or more, preferably 90 wt% or more, more preferably 97 wt% or more of water based on the total weight of water contained in the initial 1,4-cyclohexanedimethanol crude composition can be removed.

Next, a first by-product removal step is performed.

The first by-product removal step is to remove a by-product having a lower boiling point than 1,4-cyclohexanedimethanol (hereinafter, referred to as low-boiling point by-product) from the 1,4-cyclohexanedimethanol composition that has undergone the water removal step.

The low-boiling point by-product is a compound having a boiling point lower than 286 °C, which is the boiling point of 1,4-cyclohexanedimethanol. For example, it may be cyclohexylmethanol (boiling point of about 181 °C), 4-methyl-1-cyclohexanemethanol (boiling point of about 197 °C), 4-methyl cyclohexane carboxylic acid (boiling point of about 134 °C), 4-methyl-3-cyclohexene-1-methanol (boiling point of about 197 °C), 4-hydroxymethyl cyclohexane carboxylic acid (boiling point of about 278-282 °C), 4-hydroxymethyl-4-cyclohexanemethanol (boiling point of about 278-282 °C), 3-hydroxymethyl-4-cyclohexanemethanol (boiling point of about 278-282 °C), or the like.

For example, the first by-product removal step is a step of distilling the 1,4-cyclohexanedimethanol composition that has undergone the water removal step in a low boiling point distillation column to separate a low-boiling point by-product to the bottom, and 1,4-cyclohexanedimethanol to the top.

At this time, the first by-product removal step may be performed by setting the temperature to 40 to 55 °C and the pressure to -1 to 1 barg at the top of the low boiling point distillation column; and the temperature to 200 to 220 °C, and the pressure to -1 to 1 barg at the bottom.

If the temperature and pressure conditions are out of the above range, the removal rate of the low-boiling point by-product may be lowered.

After the first by-product removal step as described above, 99 wt% or more, preferably 99.5 wt% or more based on the total weight of the low-boiling point by-product contained in the initial 1,4-cyclohexanedimethanol crude composition can be removed.

Subsequently, a second by-product removal step is performed.

The second by-product removal step is to recover purified 1,4-cyclohexanedimethanol by removing a by-product having a higher boiling point than 1,4-cyclohexanedimethanol (hereinafter, referred to as high-boiling point by-product) from the 1,4-cyclohexanedimethanol composition that has undergone the first by-product removal step.

The high-boiling point by-product is a compound having a boiling point higher than 286 °C, which is the boiling point of 1,4-cyclohexanedimethanol. For example, it may be 1,4-cyclohexane-hydroxylmethyl-carboxylic acid (boiling point of about 316 °C), a monoester-based compound, a monoether-based compound, or the like.

For example, the second by-product removal step is a step of distilling the 1,4-cyclohexanedimethanol composition that has undergone the first by-product removal step in a high boiling point distillation column to separate a high-boiling point by-product to the top, and 1,4-cyclohexanedimethanol to the bottom.

At this time, the second by-product removal step may be performed by setting the temperature to 200 to 220 °C and the pressure to -1 to 1 barg at the top of the high boiling point distillation column; and the temperature to 240 to 260 °C, and the pressure to -1 to 1 barg at the bottom.

If the temperature and pressure conditions are out of the above range, the removal rate of the high-boiling point by-product may be lowered.

After the second by-product removal step as described above, 99 wt% or more, preferably 99.5 wt% or more based on the total weight of the high-boiling point by-product contained in the initial 1,4-cyclohexanedimethanol crude composition can be removed.

The 1,4-cyclohexanedimethanol composition obtained by the purification method of the present disclosure including the above-described water removal step, first by-product removal step, and second by-product removal step may have a 1,4-cyclohexanedimethanol purity of 99.5 wt% or more, when calculated as the content of 1,4-cyclohexanedimethanol analyzed by gas chromatography (GC).

More specifically, in the purified 1,4-cyclohexanedimethanol composition, the content of 1,4-cyclohexanedimethanol, that is, the purity may be 99.5 wt% or more, 99.7 wt% or more, 99.75 wt% or more, 99.8 wt% or more, or 99.9 wt% or more.

In addition, the content of water remaining in the purified 1,4-cyclohexanedimethanol composition may be 0.15 wt% or less, 0.13 wt% or less, 0.12 wt% or less, 0.10 wt% or less, 0.09 wt% or less, 0.08 wt% or less, or 0.07 wt% or less based on the total weight of the purified 1,4-cyclohexanedimethanol composition, indicating a very low water content.

When water is included in an amount of more than 0.15 wt%, low-boiling point components are not sufficiently removed, and the purity of the final 1,4-cyclohexanedimethanol may be lowered.

In addition, the 1,4-cyclohexanedimethanol composition obtained by the purification method of the present disclosure may include a light by-product having a molecular weight of less than 144.21 g/mol, which is the molecular weight (Mw) of 1,4-cyclohexanedimethanol in an amount of 0.15 wt% or less, 0.14 wt% or less, 0.12 wt% or less, 0.10 wt% or less, or 0.09 wt% or less based on the total weight of the purified 1,4-cyclohexanedimethanol composition. As the lower content of the light by-product can be evaluated as the better, the lower limit may be 0 wt%, for example, 0.01 wt%.

In the case of producing another polymer using the 1,4-cyclohexanedimethanol composition including more than 0.15 wt% of the light by-product, it may be difficult to manufacture a high molecular weight and high quality product due to hindered polymerization of the polymer.

Examples of the light by-product may include cyclohexylmethanol, 4-methyl-1-cyclohexanemethanol, cyclohexane carboxylic acid, 4-methyl-3-cyclohexene-1-methanol, 4-methyl cyclohexane carboxylic acid, 4-hydroxymethyl cyclohexane carboxylic acid, 4-hydroxymethyl-4-cyclohexanemethanol, 3-hydroxymethyl-4-cyclohexanemethanol, and the like.

When a large amount of cyclohexylmethanol (CHM) among the above-described light by-products is included in the final product, the distribution of the low molecular weight polymer increases during polymer production, which affects intrinsic properties of the polymer such as the molecular weight distribution and intrinsic viscosity. Therefore, it is preferable to remove cyclohexylmethanol as much as possible and to include substantially no cyclohexylmethanol.

The 1,4-cyclohexanedimethanol composition obtained by the purification method of the present disclosure includes only a very small amount of 0.01 wt% or less, or 0.005 wt% or less or only an undetectable amount of the cyclohexylmethanol, so that it can be used as a raw material for high-quality polymer products.

In addition, the 1,4-cyclohexanedimethanol composition obtained by the purification method of the present disclosure includes a heavy by-product having a molecular weight of more than 144.21 g/mol, which is the molecular weight (Mw) of 1,4-cyclohexanedimethanol, in an amount of 0.15 wt% or less, 0.14 wt% or less, 0.12 wt% or less, 0.10 wt% or less, or 0.09 wt% or less based on the total weight of the purified 1,4-cyclohexanedimethanol composition. As the lower content of the heavy by-product can be evaluated as the better, the lower limit may be 0 wt%, for example, 0.01 wt%.

In the case of producing another polymer using the 1,4-cyclohexanedimethanol composition including more than 0.15 wt% of the heavy by-product, polymerization of the polymer is hindered and color, viscosity, etc. are affected, making it difficult to manufacture a high-quality product.

Examples of the heavy by-product may include 1,4-cyclohexane-hydroxylmethyl-carboxylic acid, a monoester-based compound, a monoether-based compound, or the like.

In addition, the 1,4-cyclohexanedimethanol composition obtained by the purification method of the present disclosure may have an APHA value of 10 or less, 8 or less, 6 or less, 4 or less, or 2 or less, when measured according to ASTM D1209, indicating excellent color.

In addition, in the 1,4-cyclohexanedimethanol composition obtained by the purification method of the present disclosure, the 1,4-cyclohexanedimethanol may contain the trans isomer, i.e., trans 1,4-cyclohexanedimethanol in an amount of 63 wt% or more, 65 wt% or more, 67 wt% or more, 69 wt% or more, or 70 wt% or more, indicating a very high trans isomer content. Further, there is no upper limit on the trans isomer ratio, but it may be, for example, 99 wt% or less, 95 wt% or less, 90 wt% or less, or 85 wt% or less.

As described above, since the 1,4-cyclohexanedimethanol obtained by the purification method of the present disclosure has a high 1,4-cyclohexanedimethanol purity and a very low content of residual water and by-products, it can be usefully used as a raw material for manufacturing a high-quality product such as pharmaceuticals, synthetic resins, synthetic fibers, or dyes.

The 1,4-cyclohexanedimethanol crude composition, which is the subject of the purification method of the present disclosure, may be prepared by a first hydrogenation reaction to 1,4-cyclohexane dicarboxylic acid (CHDA) using terephthalic acid as a starting material, followed by a second hydrogenation reaction from 1,4-cyclohexyndicarboxylic acid to 1,4-cyclohexanedimethanol.

For example, it may be prepared by a preparation method including the following first step and the following second step. The first step is as follows: a reaction solution containing terephthalic acid, a first hydrogenation catalyst, and water, and hydrogen gas are supplied to a first reactor equipped with an agitator, and a hydrogenation reaction is performed to obtain 1,4-cyclohexane dicarboxylic acid (CHDA) containing cis isomers and trans isomers. The second step is as follows: a reaction solution containing the first reaction product, a second hydrogenation catalyst, and water, and hydrogen gas are supplied to a second reactor equipped with an agitator, and a hydrogenation reaction is performed to obtain 1,4-cyclohexanedimethanol (CHDM) containing cis isomers and trans isomers.

In the first step, a reaction solution containing terephthalic acid, a first hydrogenation catalyst, and water, and hydrogen gas are supplied to a first reactor equipped with an agitator, and a hydrogenation reaction is performed to obtain 1,4-cyclohexane dicarboxylic acid containing cis isomers and trans isomers.

More specifically, a reaction solution containing terephthalic acid, a first hydrogenation catalyst, and water is supplied to a first reactor equipped with an agitator.

The terephthalic acid is included in an amount of 5 to 25 wt% based on the total amount of terephthalic acid and water. More specifically, the terephthalic acid may be included in an amount of 5 wt% or more, 10 wt% or more, 15 wt% or more, or 18 wt% or more, and 25 wt% or less, 24 wt% or less, or 22 wt% or less, based on the total amount of terephthalic acid and water.

When the terephthalic acid is included in an amount of less than 5 wt% based on the total amount of terephthalic acid and water, the trans/cis isomer ratio takes a very long time to reach equilibrium, so there is a problem in that the trans isomer ratio in CHDA to be produced is low. When it exceeds 25 wt%, low solubility of terephthalic acid makes dissolution difficult, and there is a problem in that the reaction temperature must be set high. When the reaction temperature is increased, a large amount of low-boiling point by-products are generated, resulting in a low yield, and catalytic activity is lowered due to thermal fatigue.

According to an embodiment of the present disclosure, a catalyst known to be used in the hydrogenation reaction of terephthalic acid may be used as the first hydrogenation catalyst.

According to an embodiment of the present disclosure, the first hydrogenation catalyst may contain at least one metal selected from the group consisting of palladium (Pd), rhodium (Rh), ruthenium (Ru), and platinum (Pt) as an active component. Preferably, the first hydrogenation catalyst may contain palladium (Pd) as an active component.

The first hydrogenation catalyst may be used by being supported on a support, and in this case, a support known in the art may be used without limitation. Specifically, a support such as carbon, zirconia ($ZrO_2$), titania ($TiO_2$), alumina ($Al_2O_3$), or silica ($SiO_2$) may be used.

Subsequently, hydrogen gas is supplied to the first reactor into which the reaction solution is added.

The hydrogenation reaction may be performed in a liquid or gas phase. According to an embodiment of the present disclosure, the hydrogenation reaction may be performed with the terephthalic acid in a liquid phase dissolved in a solvent such as water, and hydrogen in a gaseous state.

Subsequently, 1,4-cyclohexane dicarboxylic acid is prepared by performing a hydrogenation reaction by stirring the agitator of the first reactor.

The reaction product obtained after the first step reaction includes CHDA containing cis isomers and trans isomers, water as a solvent, catalyst, etc., and will be used as a reactant for the second hydrogenation reaction (hydrogenation reaction from CHDA to CHDM). If necessary, the catalyst contained in the reaction product may be removed by a catalyst filter or the like, and then sent to the reaction product of the second hydrogenation reaction.

According to an embodiment of the present disclosure, the total 1,4-cyclohexane dicarboxylic acid containing cis isomers and trans isomers in the first step reaction product may be included in an amount of 5 to 30 wt% of the total amount of 1,4-cyclohexane dicarboxylic acid and water. More specifically, it may be included in an amount of 5 wt% or more, 7 wt% or more, or 10 wt% or more, and 30 wt% or less, 25 wt% or less, or 23 wt% or less.

According to an embodiment of the present disclosure, when producing 1,4-cyclohexane dicarboxylic acid by hydrogenating a mixed solution containing 5 to 25 wt%, preferably 10 to 25 wt%, more preferably 12 to 22 wt% of terephthalic acid based on the total amount of terephthalic acid and water in the mixed solution containing terephthalic acid, a first hydrogenation catalyst and water, a trans isomer ratio in the produced 1,4-cyclohexane dicarboxylic acid may be 60 wt% or more, 62 wt% or more, 65 wt% or more, 67 wt% or more, or 70 wt% or more. There is no upper limit on the trans isomer ratio, but it may be, for example, 80 wt% or less, 78 wt% or less, or 75 wt% or less.

In the second step, a reaction solution containing the reaction product of the first step, a second hydrogenation catalyst, and water, and hydrogen gas are supplied to a second reactor equipped with an agitator, and a hydrogenation reaction is performed to obtain 1,4-cyclohexanedimethanol (CHDM) containing cis isomers and trans isomers.

More specifically, a reaction solution containing the reaction product of the first step, a second hydrogenation catalyst, and water is supplied to a second reactor equipped with an agitator.

The reaction product of the first step includes 1,4-cyclohexane dicarboxylic acid, the first hydrogenation catalyst, and water as a solvent, which can be used as a reactant for the second hydrogenation reaction. At this time, it is preferable to remove the first hydrogenation catalyst contained in the reaction product of the first step with a filter, or the like before performing the second step.

In addition, the first step reaction product contains water as a solvent in addition to 1,4-cyclohexane dicarboxylic acid, so that it can be used in the second step reaction as it is without adding additional water. Alternatively, if necessary to adjust the concentration of the reaction solution, some water may be removed or additionally added.

The 1,4-cyclohexane dicarboxylic acid is included in an amount of 5 to 30 wt% based on the total amount of 1,4-cyclohexane dicarboxylic acid and water. More specifically, it may be included in an amount of 5 wt% or more, 7 wt% or more, or 10 wt% or more, and 30 wt% or less, 25 wt% or less, or 23 wt% or less.

When the 1,4-cyclohexane dicarboxylic acid is included in an amount of less than 5 wt% based on the total amount of 1,4-cyclohexane dicarboxylic acid and water, the reaction rate may be lowered due to a decrease in contact between the reactant and the catalyst, or the trans isomer ratio in the resulting CHDM may decrease. When exceeding 30 wt%, the solubility of 1,4-cyclohexane dicarboxylic acid is lowered, resulting in a decrease in productivity, and accordingly, the crystallinity of reactants and the amount of catalysts increase, which may cause difficulties in the slurry feeding process.

Herein, the ratio of cis isomer and trans isomer of 1,4-cyclohexane dicarboxylic acid, which is a reaction raw material, is the same as the ratio of cis isomer and trans isomer of 1,4-cyclohexane dicarboxylic acid obtained in the first hydrogenation reaction. Accordingly, the 1,4-cyclohexane dicarboxylic acid may have the trans isomer ratio of 60 wt% or more, 62 wt% or more, 65 wt% or more, 67 wt% or more, or 70 wt% or more. There is no upper limit on the trans isomer ratio, but it may be, for example, 80 wt% or less, 78 wt% or less, or 75 wt% or less.

According to an embodiment of the present disclosure, the second hydrogenation catalyst may include at least one metal selected from the group consisting of palladium (Pd), rhodium (Rh), and ruthenium (Ru) as an active component, and at least one metal selected from the group consisting of tin (Sn), iron (Fe), rhenium (Re), and gallium (Ga).

Preferably, ruthenium (Ru) and tin (Sn) may be included as an active component of the second hydrogenation catalyst. More preferably, the active component of the hydrogenation catalyst consists only of ruthenium (Ru) and tin (Sn), and may not include other active components.

The second hydrogenation catalyst may be used by being supported on a support, and in this case, a support known in the art may be used without limitation. Specifically, a support such as carbon, zirconia ($ZrO_2$), titania ($TiO_2$), alumina ($Al_2O_3$), or silica ($SiO_2$) may be used.

Subsequently, hydrogen gas is supplied to the reactor into which the reaction solution is added.

The hydrogenation reaction may be performed in a liquid or gas phase. According to an embodiment of the present disclosure, the hydrogenation reaction may be performed with the 1,4-cyclohexane dicarboxylic acid in a liquid phase dissolved in a solvent such as water, and hydrogen in a gaseous state.

Subsequently, 1,4-cyclohexanedimethanol is prepared by performing a hydrogenation reaction by stirring the agitator of the second reactor.

The 1,4-cyclohexanedimethanol crude composition, which is a reaction product obtained after the second step reaction, includes 1,4-cyclohexanedimethanol containing cis isomers and trans isomers, water as a solvent, a reaction by-product, etc., and is purified by the purification method of the present disclosure to obtain high-purity 1,4-cyclohexanedimethanol.

According to an embodiment of the present disclosure, the total amount of 1,4-cyclohexanedimethanol containing cis isomers and trans isomers may be 10 to 40 wt%, or 15 to 30 wt% of the total weight of the step reaction product.

Preferably, in the 1,4-cyclohexanedimethanol, which is the reaction product of the second step, the trans isomer content may be 63 wt% or more, 65 wt% or more, 67 wt% or more, 69 wt% or more, or 70 wt% or more, indicating a very high trans isomer content. Further, there is no upper limit on the trans isomer ratio, but it may be, for example, 99 wt% or less, 95 wt% or less, 90 wt% or less, or 85 wt% or less.

As described above, 1,4-cyclohexanedimethanol obtained after the second hydrogenation reaction has a high trans isomer content, so it can be usefully used as a raw material for producing higher quality products without an additional isomerization process, and can be purified by the purification method according to an embodiment of the present disclosure to recover high-purity 1,4-cyclohexanedimethanol.

The present invention will be described in more detail in order to aid understanding of the present invention. However, these examples are for illustrative purposes only, and the invention is not intended to be limited by these examples.

**<Examples>**

**Preparation Example 1**

The step 1

A first reactor equipped with a gas-induced type agitator was prepared.

550 g of terephthalic acid (TPA) as a reactant, 92 g of a 5 wt% hydrogenation catalyst Pd/C (containing 5 wt% of Pd relative to carbon support), and 2,100 g of distilled water as a solvent were added to the reactor, and the atmosphere inside the reactor was replaced with nitrogen. Thereafter, the temperature of the mixed solution was raised to 250 °C while stirring at 50 rpm.

After the temperature of the mixed solution reached 250 °C, stirring was performed for 30 minutes while maintaining the temperature for dissolution of TPA. Thereafter, the hydrogenation reaction was performed for 1 hour while increasing the stirring speed and supplying hydrogen gas into the reaction solution so that the inside of the reactor maintained at 120 bar and a surface area per unit volume of hydrogen gas maintained 300 to 500 $m^2/m^3$.

After completion of the reaction, a product containing 569 g of 1,4-cyclohexanedimethanol (CHDA) (trans CHDA ratio in CHDA: 68 wt%) and 2,100 g of water was obtained. After removing only the hydrogenation catalyst with a metal filter, it was used as a reactant in the second hydrogenation step.

The second step

A second reactor equipped with a gas-induced type agitator was prepared.

In the second reactor, 569 g of CHDA (trans CHDA ratio in CHDA: 68 wt%) which is the product of the first step reaction, and 2,100 g of distilled water as a solvent were added to 152 g of a catalyst (ruthenium-tin/carbon catalyst, containing 5 parts by weight of ruthenium and 5.8 parts by weight of tin based on 100 parts by weight of carbon support), purged twice with nitrogen at 5 bar and twice with hydrogen at 5 bar, and then stirred at 50 rpm in a hydrogen atmosphere (about 14 to 15 bar) while raising the temperature to 230 °C.

When the reaction temperature was reached, hydrogen was injected up to the reaction pressure of 100 bar, the stirring speed was increased, and the reaction was performed for 6 hours by maintaining 300 to 450 $m^2/m^3$ of the surface area per unit volume of hydrogen gas bubbles.

The hydrogenation catalyst was removed from the product of the second step reaction with a metal filter, and then transferred to a purification step of Example.

### Example 1

The following steps were performed on the 1,4-cyclohexanedimethanol crude composition of Preparation Example 1.

First, water was evaporated from the crude product in an evaporator at an operating temperature of 103 °C and a pressure of 0 barg to remove up to 97.5 wt% of the initial water content.

A first by-product removal step of separating low-boiling point by-products was performed on the 1,4-cyclohexanedimethanol composition that had undergone the water removal step by distillation in the distillation column under the following conditions: the operating pressure was -0.91 barg, and the operating temperature was 45 °C at the top; and the operating pressure was -0.9 barg, and the operating temperature was 213 °C at the bottom.

The 1,4-cyclohexanedimethanol composition that had undergone the first by-product removal step was distilled in the distillation column under the following conditions to separate a high-boiling point by-product and obtain purified 1,4-cyclohexanedimethanol: the operating pressure was -0.91 barg, and the operating temperature was 210 °C at the top; and the operating pressure was - 0.9 barg, and the operating temperature was 250 °C at the bottom.

### Example 2

A 1 ,4-cyclohexanedimethanol crude composition was purified in the same manner as in Example 1, except that the operating temperature at the top was 205 °C in the separation step of a high-boiling point by-product of Example 1.

### Comparative Example 1

The 1,4-cyclohexanedimethanol composition in which only the water removal step was performed in Example 1 was used as Comparative Example 1.

### Comparative Example 2

The 1,4-cyclohexanedimethanol crude composition of Preparation Example 1 was used as Comparative Example 2.

### Comparative Example 3

Commercialized 1,4-cyclohexanedimethanol (product name: SKY CHDM, manufacturer: SK Chemicals Co., Ltd.) was used as Comparative Example 3.

### Comparative Example 4

Commercialized 1,4-cyclohexanedimethanol (product name: CHDM-D, manufacturer: Eastman Chemical Company) was used as Comparative Example 4.

### <Experimental Example>

The purity, content of light by-products and heavy by-products, water content, trans-CHDM content in CHDM, and APHA value of the 1,4-cyclohexanedimethanol compositions of Examples and Comparative Examples were measured by the following methods and shown in Table 2.

1) Purity (total content of CHDM), content of light by-products and heavy by-products, content of trans-CHDM in CHDM

They were measured by Gas Chromatography, and detailed conditions are shown in Table 1 below.

[Table 1]

| Instrument | HP-6890 Series GC System |
|---|---|
| Sample preparation | Sample(CHDM) 0.2g + pyridine 4ml Silylation sample |
| Sample | 1 μL |

(continued)

| Instrument | | | HP-6890 Series GC System | |
|---|---|---|---|---|
| Detector | | FID | FID | |
| | | Temp | 300 °C | |
| | | H2 | 40 ml/min | |
| | | Make Up(He) | 30 ml/min | |
| | | Air | 350 ml/min | |
| Injection | | Temp | 300 °C | |
| | | Injection Mode | Split (ratio 1:75) | |
| | | Control Mode | Pressure/16.51 psi | |
| | | Carrier Gas | He | |
| | | Column Flow | 3.0 ml/min | |
| Column | | | HP-5 (5%-phenyl-methylpolysiloxane capillary column) 30.0 m X 0.32 mm(ID) X 0.25μm(Film) | |
| Oven | | Rate | Temp. | Hold |
| Temp. program | - | 115 °C | 0 min | |
| | 5 °C/min | 130 °C | 0 min | |
| | 10 °C/min | 200 °C | 0 min | |
| | 12 °C/min | 280 °C | 4 min | |
| Calculation | | Area% Component $= \dfrac{(\text{Area of Component}) \times 100}{\text{Sum of Areas of All Detected Components}}$ (Excluding pyridine Solvent) | | |

2) Water content

It was measured according to ASTM D1364 (karl Fischer).

3) APHA value

It was measured according to ASTM D1209.

[Table 2]

| | Ex. 1 | Ex.2 | Comp. Ex. 1 | Comp. Ex.2 | Comp. Ex. 3 | Comp. Ex.4 |
|---|---|---|---|---|---|---|
| CHDM purity (wt%) | 99.74 | 99.71 | 87.64 | 12.2 | 99.42 | 99.24 |
| Light by-product (wt%) | 0.07 | 0.07 | 1.18 | 0.2 | 0.20 | 0.30 |
| Heavy by-product (wt%) | 0.13 | 0.13 | 2.09 | 0.3 | 0.20 | 0.20 |
| CHM content (wt%) | 0.001 | 0.001 | 3.0 | 0.26 | 0.014 | 0.013 |
| Water (wt%) | 0.06 | 0.09 | 9.09 | 87.3 | 0.18 | 0.26 |
| Trans CHDM content (wt%) | 75 | 76 | 75 | 75 | 66 | 69 |
| APHA value | 1.8 | 1.8 | 100 | 75 | 2.9 | 2.9 |

Referring to Table 2, the 1,4-cyclohexanedimethanol compositions according to the purification methods of Examples 1 and 2 of the present disclosure showed a very low by-product content of less than 0.15 wt% in both light and heavy by-products.

In addition, the content of CHM (cyclohexylmethanol), which is a by-product adversely affecting when used as a polymer raw material, was very low as 0.001 wt%, so that high-quality 1,4-cyclohexanedimethanol can be provided.

**Claims**

1. A 1,4-cyclohexanedimethanol composition comprising 1,4-cyclohexanedimethanol (CHDM) having a purity of 99.5 wt% or more as measured by gas chromatography (GC) and containing cis isomers and trans isomers; and 0.15 wt% or less of a light by-product having a molecular weight of less than 144.21 g/mol.

2. The 1,4-cyclohexanedimethanol composition of Claim 1, comprising 0.01 wt% or less of cyclohexylmethanol.

3. The 1,4-cyclohexanedimethanol composition of Claim 1, comprising 0.15 wt% or less of a heavy by-product having a molecular weight of more than 144.21 g/mol.

4. The 1,4-cyclohexanedimethanol composition of Claim 1, wherein an APHA value measured according to ASTM D1209 is 10 or less.

5. The 1,4-cyclohexanedimethanol composition of Claim 1, wherein the 1,4-cyclohexanedimethanol contains 63 wt% or more of trans isomers.

6. A method for purifying a 1,4-cyclohexanedimethanol composition, comprising the steps of:

   a water removal step of removing water from a 1,4-cyclohexanedimethanol (CHDM) crude composition;
   a first by-product removal step of removing a by-product having a lower boiling point than 1,4-cyclohexanedimethanol from the 1,4-cyclohexanedimethanol composition that has undergone the water removal step; and
   a second by-product removal step of recovering a purified 1,4-cyclohexanedimethanol composition by removing a by-product having a higher boiling point than 1,4-cyclohexanedimethanol from the 1,4-cyclohexanedimethanol composition that has undergone the first by-product removal step.

7. The method for purifying a 1,4-cyclohexanedimethanol composition of Claim 6, wherein the water removal step is performed at a temperature of 50 to 110 °C and a pressure of -0.1 to 0.1 barg.

8. The method for purifying a 1,4-cyclohexanedimethanol composition of Claim 6, wherein the 1,4-cyclohexanedimethanol crude composition comprises 1,4-cyclohexanedimethanol, water, and a by-product.

9. The method for purifying a 1,4-cyclohexanedimethanol composition of Claim 6, wherein the 1,4-cyclohexanedimethanol has a purity of 99.5 wt% or more in the purified 1,4-cyclohexanedimethanol composition.

10. The method for purifying a 1,4-cyclohexanedimethanol composition of Claim 6, wherein the purified 1,4-cyclohexanedimethanol composition comprises 0.15 wt% or less of water.

11. The method for purifying a 1,4-cyclohexanedimethanol composition of Claim 6, wherein the purified 1,4-cyclohexanedimethanol composition comprises 0.15 wt% or less of a light by-product having a molecular weight of less than 144.21 g/mol.

12. The method for purifying a 1,4-cyclohexanedimethanol composition of Claim 6, wherein the purified 1,4-cyclohexanedimethanol composition comprises 0.15 wt% or less of a heavy by-product having a molecular weight of more than 144.21 g/mol.

13. The method for purifying a 1,4-cyclohexanedimethanol composition of Claim 6, wherein the purified 1,4-cyclohexanedimethanol composition has an APHA value measured according to ASTM D1209 of 10 or less.

14. The method for purifying a 1,4-cyclohexanedimethanol composition of Claim 6, wherein the 1,4-cyclohexanedimethanol contains 63 wt% or more of trans isomers.

## INTERNATIONAL SEARCH REPORT

| International application No. |
|---|
| **PCT/KR2022/001257** |

### A. CLASSIFICATION OF SUBJECT MATTER

**C07C 31/27**(2006.01)i; **C07C 29/32**(2006.01)i; **C07C 29/149**(2006.01)i; **B01J 23/44**(2006.01)i

According to International Patent Classification (IPC) or to both national classification and IPC

### B. FIELDS SEARCHED

Minimum documentation searched (classification system followed by classification symbols)

C07C 31/27(2006.01); B01D 11/04(2006.01); B01J 23/44(2006.01); B01J 8/02(2006.01); C07C 29/147(2006.01); C07C 29/149(2006.01); C07C 29/17(2006.01); C07C 51/36(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 사이클로헥산디메탄올(cyclohexanedimethanol, CHDM), 정제(purification), 물(water), 끓는점(boiling point)

### C. DOCUMENTS CONSIDERED TO BE RELEVANT

| Category* | Citation of document, with indication, where appropriate, of the relevant passages | Relevant to claim No. |
|---|---|---|
| Y | KR 10-2014-0058584 A (EASTMAN CHEMICAL COMPANY) 14 May 2014 (2014-05-14) <br> See claim 1; paragraph [0219]; and table 13, cut 3. | 1-14 |
| Y | KR 10-2020-0044109 A (CLEARWATERBAY CHDM TECHNOLOGY LIMITED et al.) 28 April 2020 (2020-04-28) <br> See claims 1 and 2; paragraph [0050]; and figure 1. | 1-14 |
| Y | KR 10-2018-0047255 A (LG CHEM, LTD.) 10 May 2018 (2018-05-10) <br> See claim 8; and paragraphs [0012], [0038] and [0039]. | 1-14 |
| A | KR 10-2019-0063704 A (LOTTE CHEMICAL CORPORATION) 10 June 2019 (2019-06-10) <br> See entire document. | 1-14 |
| A | KR 10-2020-0081096 A (HANWHA SOLUTIONS CORPORATION) 07 July 2020 (2020-07-07) <br> See entire document. | 1-14 |

☐ Further documents are listed in the continuation of Box C.    ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **13 May 2022** | **13 May 2022** |

| Name and mailing address of the ISA/KR | Authorized officer |
|---|---|
| **Korean Intellectual Property Office** <br> **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**
Information on patent family members

International application No.

**PCT/KR2022/001257**

| Patent document cited in search report | | | Publication date (day/month/year) | Patent family member(s) | | | Publication date (day/month/year) |
|---|---|---|---|---|---|---|---|
| KR | 10-2014-0058584 | A | 14 May 2014 | BR | 112014001769 | A2 | 21 February 2017 |
| | | | | CN | 103687833 | A | 26 March 2014 |
| | | | | CN | 103687833 | B | 29 June 2016 |
| | | | | EP | 2736868 | A1 | 04 June 2014 |
| | | | | EP | 2736868 | B1 | 25 November 2015 |
| | | | | JP | 2014-527521 | A | 16 October 2014 |
| | | | | JP | 5973565 | B2 | 23 August 2016 |
| | | | | TW | 201309631 | A | 01 March 2013 |
| | | | | TW | I545107 | B | 11 August 2016 |
| | | | | US | 2013-0030219 | A1 | 31 January 2013 |
| | | | | US | 8410317 | B2 | 02 April 2013 |
| | | | | WO | 2013-019439 | A1 | 07 February 2013 |
| KR | 10-2020-0044109 | A | 28 April 2020 | CN | 111263745 | A | 09 June 2020 |
| | | | | US | 10329235 | B2 | 25 June 2019 |
| | | | | US | 2019-0062251 | A1 | 28 February 2019 |
| | | | | WO | 2019-046412 | A1 | 07 March 2019 |
| KR | 10-2018-0047255 | A | 10 May 2018 | KR | 10-2224243 | B1 | 08 March 2021 |
| KR | 10-2019-0063704 | A | 10 June 2019 | None | | | |
| KR | 10-2020-0081096 | A | 07 July 2020 | CN | 113056446 | A | 29 June 2021 |
| | | | | EP | 3904323 | A1 | 03 November 2021 |
| | | | | JP | 2022-513714 | A | 09 February 2022 |
| | | | | KR | 10-2021-0154130 | A | 20 December 2021 |
| | | | | WO | 2020-138973 | A1 | 02 July 2020 |

Form PCT/ISA/210 (patent family annex) (July 2019)

**EP 4 289 808 A1**

**REFERENCES CITED IN THE DESCRIPTION**

**Patent documents cited in the description**

- KR 1020210015384
- KR 1020210015385